**Europäisches Patentamt**

**European Patent Office**

**Office européen des brevets**

(19)

(11) Veröffentlichungsnummer : **0 416 415 B1**

(12) # EUROPÄISCHE PATENTSCHRIFT

(45) Veröffentlichungstag der Patentschrift :
**21.07.93 Patentblatt 93/29**

(51) Int. Cl.$^5$ : **C01G 30/00, C22B 7/00**

(21) Anmeldenummer : **90116365.9**

(22) Anmeldetag : **27.08.90**

(54) **Verfahren zur Aufarbeitung von Antimonhalogenidkatalysatorlösungen.**

(30) Priorität : **02.09.89 DE 3929263**

(43) Veröffentlichungstag der Anmeldung :
**13.03.91 Patentblatt 91/11**

(45) Bekanntmachung des Hinweises auf die
Patenterteilung :
**21.07.93 Patentblatt 93/29**

(84) Benannte Vertragsstaaten :
**AT BE CH DE DK ES FR GB IT LI NL SE**

(56) Entgegenhaltungen :
**US-A- 3 787 557**
**US-A- 4 722 774**

(73) Patentinhaber : **Kali-Chemie
Aktiengesellschaft
Postfach 220, Hans-Böckler-Allee 20
W-3000 Hannover 1 (DE)**

(72) Erfinder : **Fernschild, Günter
Am Asphaltberge 8
verstorben (DE)**

(74) Vertreter : **Lauer, Dieter, Dr.
c/o Kali-Chemie Aktiengesellschaft Postfach
220
W-3000 Hannover 1 (DE)**

**Beschreibung**

Die Erfindung betrifft ein Verfahren zur Aufarbeitung von inaktiven, Antimonhalogenidkatalysatoren und organische Verunreinigungen enthaltenden Lösungen.

Fluorchlorkohlenwasserstoffe, Fluorchlorkohlenstoffe, Fluorkohlenwasserstoffe und Fluorkohlenstoffe werden in großem Umfang als Lösungs- und Kältemittel verwendet (der Einfachheit halber wird im folgenden nur noch von Kohlenwasserstoffverbindungen die Rede sein. Unter dieser Bezeichnung werden in der vorliegenden Erfindung auch wasserstofffreie Verbindungen verstanden, die korrekterweise als "Kohlenstoffverbindungen" bezeichnet werden mußten. Der Begriff "Fluorchlorkohlenwasserstoff" umfaßt also beispielsweise auch $CFCl_3$). Bei den meisten großtechnischen Herstellverfahren erfolgt ein durch Antimonhalogenid katalysierter Chlor-Fluor-Austausch. Als Katalysator wird Antimonpentahalogenid, insbesondere Antimonpentachlorid, eingesetzt.

Die Lebensdauer dieser Katalysatoren ist beschränkt. Während der Herstellung von Fluorchlorkohlenwasserstoffen bzw. Fluorkohlenwasserstoffen bilden sich neben den gewünschten Verfahrensprodukten auch unerwünschte, halogenierte organische Verbindungen, von denen einige beträchtlich höhere Siedepunkte als die gewünschten Produkte besitzen. Diese Nebenprodukte verdünnen den Antimonhalogenidkatalysator, so daß sich allmählich Antimonhalogenidkatalysatoren und organische Verunreinigungen enthaltende Lösungen mit zunehmend nachlassender Aktivität bilden. Wenn die nachlassende Aktivität der Katalysatoren eine bestimmte Grenze unterschreitet, muß die inaktive Katalysatorlösung aus dem Reaktor entfernt und durch frischen Antimonhalogenidkatalysator ersetzt werden.

Die Lösungen enthalten fünfwertiges und gewöhnlich auch dreiwertiges Antimon in Form der Halogenide, und zwar vorwiegend in Form der Chloride, in mehr oder weniger geringem Umfang auch in Form von gemischten Chlorid-Fluoriden. Je nach Einsatzgebiet der Katalysatorlösung kann der Fluoridgehalt auch sehr gering sein. Neben den anorganischen Bestandteilen sind in den Lösungen weiterhin halogenierte Kohlenwasserstoffe vorhanden. Welche speziellen halogenierten Kohlenwasserstoffe jeweils vorhanden sind, ist natürlich vom jeweiligen Fluorierungsverfahren, bei dem die Katalysatorlösung eingesetzt worden war, und der Art seiner Durchführung abhängig.

Die Aufarbeitung der inaktiven Lösungen ist aus ökonomischen Gründen (Rückgewinnung des Antimongehalts), besonders aber auch aus ökologischen Gründen wünschenswert.

Antimonpentahalogenide und die halogenierten Kohlenwasserstoffe der inaktiven Lösung bilden ein homogenes Gemisch. Da einige der organischen Bestandteile einen ähnlichen Siedepunkt wie beispielsweise Antimonpentachlorid besitzen, ist die destillative Aufarbeitung nicht möglich. Aufgrund der Bildung homogener Phasen und der ähnlichen Siedepunkte von Antimonhalogeniden und Verunreinigungen ist die Abtrennung der Antimonhalogenide von den organischen Bestandteilen bei der Aufarbeitung mit Schwierigkeiten verbunden.

Es sind bereits Verfahren zur Aufarbeitung von inaktiven, Antimonhalogenidkatalysatoren und organische Verunreinigungen enthaltenden Lösungen bekannt.

Eine Vorgehensweise sieht die wässrige Aufarbeitung vor. Nach dem Verfahren der DE-A-25 45 220 versetzt man die Antimonhalogenidkatalysatoren enthaltende organische Lösung zur Abtrennung des Antimonhalogenids von den organischen Bestandteilen mit soviel Wasser oder wässriger Salzsäure, daß sich zwei flüssige Phasen bilden. Eine Phase wird durch die organischen Bestandteile gebildet, die andere, wässrige Phase enthält die Anteile an fünfwertigem Antimon. Die hydratisierten Antimonverbindungen können nun leicht von den organischen Bestandteilen getrennt und gewünschtenfalls reduziert werden. Die Überführung der gelösten Antimonverbindungen in wasserfreie Verbindungen, insbesondere in die wasserfreien Antimonhalogenide, ist allerdings sehr umständlich. Deshalb werden die wässrigen, nach Reduzieren erhaltenen Antimontrihalogenidlösungen unmittelbar zur Herstellung von Bleichromatpigmenten verwendet. Nach dem Verfahren der US-A-47 22 774 wird elementares Antimon zu einer inaktiven, Antimonpentachlorid enthaltenden organischen Lösung zugegeben; das gebildete $SbCl_3$ wird auch mit wässriger Salzsäure extrahiert und dann abgetrennt.

Eine andere Vorgehensweise sieht die nichtwässrige Aufarbeitung vor. Ein solches Verfahren wird in der DE-A-20 56 648 beschrieben. Dort wird die Antimonhalogenidkatalysatoren und organische Verunreinigungen enthaltende Lösung mit dem gleichen Volumen Trichlorethylen versetzt und das Gemisch im Autoklaven erhitzt. Das gebildete, kristallin und wasserfrei anfallende Antimontrichlorid läßt sich durch Abnutschen von den organischen Bestandteilen abtrennen und gewünschtenfalls zu Antimonpentachlorid aufarbeiten, das erneut als Katalysator verwendbar ist. Die bei diesem Verfahren notwendige Zugabe einer erheblichen Menge an Trichlorethylen (andere Olefine, außer Perchlorethylen, erwiesen sich als unbrauchbar) führt jedoch zu entsprechend höherem Anfall an unerwünschten hochsiedenden Nebenprodukten.

Aufgabe der vorliegenden Erfindung ist es, ein verbessertes Verfahren zur Aufarbeitung von inaktiven, Antimonhalogenidkatalysatoren und organische Verunreinigungen enthaltenden organischen Lösungen anzugeben, welches die Nachteile der aus dem Stand der Technik bekannten Verfahren überwindet.

Diese Aufgabe wird durch das erfindungsgemäße Verfahren gelöst.

Das erfindungsgemäße Verfahren zur Aufarbeitung von inaktiven, Antimonhalogenidkatalysatoren und organische Verunreinigungen enthaltenden organischen Lösungen ohne Zusatz von Wasser ist dadurch gekennzeichnet, daß man zu dieser Lösung elementares Antimon zufügt und die organischen Verunreinigungen vom gebildeten kristallinen Antimontrichlorid abtrennt.

Nach dem erfindungsgemäßen Verfahren kann man inaktive, Antimonhalogenidkatalysatoren und organische Verunreinigungen enthaltende Lösungen aufarbeiten, wie sie bei der Fluorierung von halogenierten, insbesondere chlorierten Kohlenwasserstoffen mit wasserfreiem Fluorwasserstoff anfallen.

Prinzipiell ist das erfindungsgemäße Verfahren zur Aufarbeitung von organischen Verunreinigungen enthaltenen Antimonhalogenidkatalysatoren geeignet, wie sie bei der Fluorierung beliebiger Halogenkohlenwasserstoffe, beispielsweise mehr oder weniger langkettigen aliphatischen, aromatischen und araliphatischen Verbindungen, mit wasserfreiem Fluorwasserstoff anfallen.

Beispielsweise ist das erfindungsgemäße Verfahren gut geeignet zur Aufarbeitung von inaktiven, Antimonpentahalogenidkatalysator und organische Verunreinigungen enthaltenden Lösungen, wie sie bei der Fluorierung von aliphatischen chlorierten Kohlenwasserstoffen, insbesondere mit einem oder zwei Kohlenstoffatomen mittels wasserfreiem Fluorwasserstoff anfallen. Hervorragend eignet es sich für die Lösungen, die bei der entsprechenden Fluorierung von $CCl_4$ zu $CFCl_3$ oder $CF_2Cl_2$, von $CHCl_3$ zu $CHClF_2$ oder von $C_2Cl_6$ zu $C_2Cl_3F_3$ mittels wasserfreiem Fluorwasserstoff anfallen.

Derartige Lösungen enthalten anorganische und organische Verbindungen. Der größte Teil der anorganischen Verbindungen wird gewöhnlich durch Antimonpentahalogenid gebildet, welches im wesentlichen als Antimonpentachlorid vorliegt. Weiterhin ist gewöhnlich eine geringe Menge an dreiwertigem Antimon, vorwiegend in Form von Antimontrichlorid, enthalten. Es kann ferner Fluorid enthalten sein sowie Metallsalze, die aus korrodiertem Reaktormaterial stammen.

Der absolute Gehalt an organischen Verbindungen in den Katalysatorlösungen ist insbesondere von der Verwendungsdauer im jeweiligen Verfahren abhängig. Üblicherweise enthalten die im erfindungsgemäßen Verfahren aufzuarbeitenden Lösungen bis zu 50 Gew.-% organische Bestandteile. Die Zusammensetzung dieser organischen Bestandteile ist davon abhängig, in welchem Herstellverfahren die Lösung angefallen war.

Beispielsweise enthalten die bei der Herstellung von $CHClF_2$ aus $CHCl_3$ anfallenden aufzuarbeitenden Lösungen höher siedende Bestandteile wie $C_2Cl_5F$, $C_2H_2Cl_4$, $C_2Cl_6$ sowie weitere höher siedende Bestandteile "die bei unerwünschten Nebenreaktionen anfallen.

Eine Zugabe von Wasser erfolgt im erfindungsgemäßen Verfahren bei der Aufarbeitung nicht. In einer bevorzugten Ausführungsform arbeitet man unter Ausschluß von Feuchtigkeit. Hierzu kann man das erfindungsgemäße Verfahren in Vorrichtungen durchführen, die mit Absperreinrichtungen versehen sind, die zwar den Druckausgleich mit der Umgebung zulassen, den Zutritt von Feuchtigkeit aus der Umgebung aber verhindern. Derartige Absperreinrichtungen sind dem Fachmann bekannt. Geeignet sind beispielsweise übliche Trockenmittel enthaltende Gaswäscher, wobei als Trockenmittel bevorzugt Schwefelsäure verwendet wird. Gewünschtenfalls kann man auch in Inertgasatmosphäre arbeiten, wobei als Inertgas außer Stickstoff auch Edelgase oder Chlorfluorkohlenwasserstoffe oder Fluorkohlenwasserstoffe in Frage kommen.

Das erfindungsgemäße Verfahren zur Aufarbeitung von inaktiven, Antimonhalogenidkatalysatoren und organische Verunreinigungen enthaltenden Lösungen sieht vor, daß man zu dieser Lösung elementares Antimon hinzufügt.

Hierzu kann man beispielsweise das Antimon in einem Reaktor vorlegen und die Lösung in den Reaktor eindosieren. Diese Verfahrensvariante besitzt den Vorteil, daß sie technisch besonders einfach durchzuführen ist.

In einer anderen Verfahrensvariante wird die organische Lösung vorgelegt und elementares Antimon in die Lösung eindosiert. Diese Variante ist technisch aufwendiger als die vorstehend beschriebene Alternative, sie besitzt allerdings den Vorteil, daß man die Menge an elementaren Antimon auf einfache Weise bemessen und der jeweils aufzuarbeitenden Lösung quantitativ anpassen kann.

Erfolgreich durchgeführt werden kann das erfindungsgemäße Verfahren nach beiden Varianten.

Das elementare Antimon kann man in mehr oder weniger großen Stücken, bis hin zu einem Gewicht von 25 kg und mehr, einsetzen. Man kann es allerdings auch in Form von kleineren Partikeln einsetzen. Geeignet ist z.B. pulverförmiges Antimon einer Partikelgröße von 0,1 bis 1 mm. Es ist zwar möglich, das Antimon in einer (auch gegenüber Antimonhalogeniden) inerten Flüssigkeit, zum Beispiel einem fluorierten oder chlorfluorierten Kohlenwasserstoff aufzuschwemmen und als Suspension einzusetzen. Dies ist jedoch nicht notwendig und auch nicht vorteilhaft.

Aus dem hinzugefügten Antimon und dem in der aufzuarbeitenden Lösung vorliegenden Antimonpentachlorid bildet sich in einer exotherm verlaufenden Konproportionierungsreaktion durch Reduktion des fünfwertigen Antimons Antimontrichlorid. Das gebildete Antimontrichlorid kristallisiert beim Abkühlen aus der Lösung aus. Die organischen Verbindungen werden nach bekannten Methoden der Fest/Flüssig-Phasentrennung vom auskristallisierten Antimontrichlorid abgetrennt. Dies geschieht in einfacher Weise durch Dekantieren, Abnutschen oder Absaugen der flüssigen Phase.

Man kann das Hinzufügen des elementaren Antimon zu der Lösung bei einer solchen in der Lösung herrschenden Temperatur durchführen, daß während der Antimonzugabe bereits Antimontrichlorid auskristallisiert. Dies kann beispielsweise durch ständiges Kühlen der organischen Lösung oder durch sehr langsame Vereinigung der Komponenten erfolgen, so daß die Temperatur der organischen Lösung unterhalb jener Temperatur gehalten wird, bei welcher Antimontrichlorid schmilzt. Diese Vorgehensweise ist zwar möglich, aber wenig vorteilhaft. Es hat sich nämlich gezeigt, daß Antimontrichlorid auf dem elementaren Antimon als Deckschicht auskristallisiert und auf diese Weise die Weiterreaktion des noch vorhandenen Antimon verhindert.

Eine bevorzugte Ausführungsform des erfindungsgemäßen Verfahrens ist deshalb dadurch gekennzeichnet, daß, während man elementares Antimon zur organischen Lösung hinzufügt, man die Lösung bei einer solchen Temperatur hält, daß gebildetes Antimontrichlorid nicht auskristallisiert. In einer besonders bevorzugten Ausführungsform des erfindungsgemäßen Verfahrens hält man die organische Lösung nach vollständiger Zugabe des elementaren Antimons während der Reaktionsphase und gewünschtenfalls auch während einer Nachreaktionsphase bei einer solchen Temperatur, daß gebildetes Antimontrichlorid nicht auskristallisiert. Eine solche Nachreaktionsphase kann bis zu 30 Stunden, bevorzugt zwischen 12 bis 24 Stunden betragen.

Die Temperatur, bei welcher man die organische Lösung halten muß, um das Auskristallisieren von Antimontrichlorid zu verhindern, kann je nach Herkunft der Lösung und der jeweils enthaltenden organischen und anorganischen Bestandteile geringfügig schwanken. Da chemisch reines Antimontrichlorid bei etwa 73°C schmilzt bzw. kristallisiert, ist es empfehlenswert, die organische Lösung während der Antimonzugabe und gegebenenfalls in einer Nachreaktionsphase bei einer Temperatur von etwa 65°C bis etwa 100°C zu halten. Gewünschtenfalls kann man aber auskristallisiertes Antimontrichlorid durch Temperaturerhöhung wieder in die flüssige Phase überführen.

Das erfindungsgemäße Verfahren kann bei einem Druck von 0,1 bis 6 Bar, vorzugsweise 0,5 bis 3 Bar durchgeführt werden.

Während das elementare Antimon der organischen Lösung zugefügt wird und gewünschtenfalls auch während einer Nachreaktionsphase ist es vorteilhaft, die Lösung intensiv zu durchmischen, beispielsweise mit einem entsprechend leistungsstarken Rührwerk.

Die einzusetzende Menge an elementarem Antimon sollte zweckmäßigerweise etwa 0,5 mol Antimon big 1 mol pro mol Antimonpentachlorid in der auf zuarbeitenden Lösung betragen. Vorteilhafterweise setzt man etwa 0,6 bis 0,9 mol, besonders bevorzugt etwa 0,7 mol bis etwa 0,8 mol elementares Antimon pro mol Antimonpentachlorid ein. Die vorzusehende Gesamtmenge an elementaren Antimon kann durch analytische Bestimmung des in der aufzuarbeitenden Lösung enthaltenen Antimonpentahalogenids festgelegt werden. Die Analyse erfolgt iodometrisch. Vorteilhafterweise überwacht man den Ablauf der Umsetzung durch mehrmaliges Entnehmen einer Probe aus der organischen Lösung und analytische Bestimmung des noch vorhandenen Gehalts an Antimonpentachlorid.

Die aufzuarbeitende Lösung wird nach beendeter Antimonzugabe und gewünschtenfalls nach einer Nachreaktionsphase auf Umgebungstemperatur, beispielsweise auf Temperaturen zwischen etwa 12° und etwa 25°C, gebracht. Dies kann durch einfaches Stehenlassen der Lösung bei Umgebungstemperatur oder auch durch Abkühlen, beispielsweise mit Kühlwasser, erfolgen. Im Verlauf des Abkühlvorgangs bildet sich kristallines Antimontrichlorid, von welchem, wie bereits beschrieben, beispielsweise durch Dekantieren, Abnutschen oder Absaugen die nunmehr im wesentlichen antimonfreie organische Phase getrennt werden kann. Das auskristallisierte Antimontrichlorid ist bereits verhältnismäßig rein (Reinheitsgrad 95 %) und kann beispielsweise chloriert und das hierbei gebildete Antimonpentachlorid erneut als Katalysator im Fluorierungsverfahren wiederverwendet werden.

In einer bevorzugten Ausführungsform unterwirft man das auskristallisierte Antimontrichlorid einer Nachreinigung. Dies kann in an sich bekannter Weise erfolgen, beispielsweise durch Auflösen in inerten Lösungsmitteln in der Hitze, Abkühlen und Abtrennen des erneut gebildeten kristallinen Antimontrichlorids vom Lösungsmittel. Gewünschtenfalls kann im Vakuum getrocknet werden. Besonders geeignet ist Tetrachlorkohlenstoff als Lösungsmittel bei dieser Nachreinigung. Das gewonnene Antimontrichlorid enthält nur noch geringe Mengen an Verunreinigung (Reinheitsgrad besser als 98 %). Gewünschtenfalls kann der Reinheitsgrad durch Wiederholung der Nachreinigung und/oder destillative Reinigung noch weiter gesteigert werden.

Das hierbei gewonnene Antimontrichlorid ist als solches verwendbar, beispielsweise als Katalysator für organische Synthesen, als Ätzmittel oder als Gerbhilfsmittel.

Vorzugsweise wird das erhaltene Antimontrichlorid mit elementaren Chlor zu Antimonpentachlorid umgesetzt. Hierzu bringt man das Antimontrichlorid bei einer Temperatur zwischen etwa 20 und etwa 100°C mit elementarem Chlor zur Reaktion. Das Chlor wird in der stöchiometrisch notwendigen Menge oder in geringem Überschuß eingesetzt, die Chlorierung erfolgt bei einem Druck zwischen 0,5 atm (abs.) und etwa 6 atm (abs.). Das erhaltene Antimonpentachlorid kann destillativ noch weiter gereinigt werden. Es kann als Katalysator für Fluorierungsreaktionen verwendet werden.

Gewünschtenfalls kann man die vorstehend beschriebene Chlorierung von Antimontrichlorid mit Chlor in Anwesenheit eines Lösungsmittels vornehmen, welches gegenüber Chlor, Antimontrichlorid und Antimonpentachlorid bei den zur Anwendung kommenden Reaktionsbedingungen inert ist. Sehr gut geeignet sind halogenierte organische Lösungsmittel wie Chloroform, Tetrachlorkohlenstoff oder Chlorfluorkohlenwasserstoffe. Das Lösungsmittel kann in einer Menge von bis zu 25 Gew.-%, bezogen auf das eingesetzte Antimontrichlorid, vorliegen.

Die nach dem Chlorieren erhältliche Lösung von Antimonpentachlorid im verwendeten Lösungsmittel ist wiederum als Katalysator für Fluorierungsreaktionen verwendbar. Hierbei ist es natürlich besonders vorteilhaft, wenn das bei der Chlorierung verwendete Lösungsmittel gleichzeitig die Ausgangsverbindung oder das Produkt des Fluorierungsverfahrens ist, in welchem das lösungsmittelhaltige Antimonpentachlorid als Katalysator verwendet werden soll. So ist Chloroform beispielsweise das vorteilhafterweise zur Anwendung kommende Lösungsmittel, wenn das in seiner Anwesenheit hergestellte Antimonpentachlorid bei der Fluorierung von Chloroform als Katalysator eingesetzt werden soll.

Das erfindungsgemäße Verfahren besitzt überraschende Vorteile:
- Die Aufarbeitung der inaktiven Antimonhalogenidkatalysatorlösungen ist unkompliziert, die Gewinnung wasserfreier Antimonhalogenide hoher Reinheit ist mit wenigen Verfahrungsstufen möglich.
- Die korrosive Beanspruchung der zum Einsatz kommenden Apparaturen ist geringer als bislang üblich.
- Es entstehen keine unerwünschten Nebenprodukte.
- Die Aufarbeitung kann im Reaktorsystem der Produktionsanlagen erfolgen.

Das folgende Beispiel soll das erfindungsgemäße Verfahren weiter erläutern, ohne seinen Schutzbereich einzuschränken.

Ausführungsbeispiel

a) Vorrichtung:

Verwendet wurde ein zylindrischer Reaktor mit einem Innenvolumen von 1050 ml und einem Innendurchmesser von 9,6 cm, dessen Reaktorwand als Doppelmantel ausgebildet war und infolgedessen flüssigkeitsgekühlt oder -erhitzt werden konnte. Er besaß eine Vorrichtung zur Zuführung eines Feststoffes, war mit einem Rührwerk und einem Kühler mit hohem Leistungsvermögen (Intensivkühler) versehen. Eine mit Schwefelsäure gefüllte Sicherheitswaschflasche gestattete den Druckausgleich mit dem umgebenden Atmosphärendruck, verhinderte aber den Zutritt von Feuchtigkeit. Etwaig entstehende Abgase wurden über einen Abgaswäscher, gefüllt mit wässriger Kalilauge, in die Umgebung entlassen.

b) Verfahrensdurchführung:

Die inaktive Antimonhalogenidkatalysatorlösung wurde zunächst analysiert. Die Antimonanalysen erfolgten maßanalytisch mit potentiometrischer Indikation (Redox-Meßkette). Titrationsmittel war n/10 (d.h. 0,1-normale) Natriumthiosulfat-Lösung und n/10 (d.h. 0,1-normale) Kaliumbromatlösung. Die Analyse ergab folgende Werte (die Zahlenangaben beziehen sich jeweils auf Gew.-%):

## Tabelle 1

| Anorg. Bestandteile | | | Organ. Bestandteile | |
| --- | --- | --- | --- | --- |
| Antimon gesamt | 25,0 | (hiervon $Sb^V$ :23,4 | Niedrigsieder ($CFCl_3$, $CF_2Cl_2$, $CHFCl_2$) | 10,7 |
| | | $Sb^{III}$: 1,6) | $CH_2Cl_2$ | 0,6 |
| Arsen | 0,2 | | $CHCl_3$ | 5,3 |
| Fluor | 1,0 | | $C_2Cl_4F_2$ | 0,7 |
| Chlor | 33,1 | | $C_2Cl_5F$ | 5,0 |
| | | | $C_2H_2Cl_4$ | 11,6 |
| | | | $C_2Cl_4$ | 0,3 |
| | | | Unidentifizierte Hochsieder | 6,5 |
| Anorg. Bestandteile gesamt: | 59,3 | | Org. Bestandteile gesamt: | 40,7 |

EP 0 416 415 B1

Die Dichte der Lösung betrug etwa 2 g/cm³.

In den Reaktor wurden 500 ml der aufzuarbeitenden organischen Lösung eingefüllt und auf etwa 65 °C erwärmt. Die Gesamtmenge des einzusetzenden Antimons betrug 183 g (etwa 1.5 mol) und war damit etwas größer, als der für die vollständige Umsetzung des analytisch bestimmten fünfwertigen Antimons stöchiometrisch notwendigen Menge entspricht (Gehalt an Antimon (V) in der Lösung: 230,5 g entsprechend 1,89 mol; stöchiometrisch notwendig: 153,7 g an elementarem Antimon).

Das elementare Antimon, das pulverförmig eingesetzt wurde, wurde aus einer gasdicht mit dem Reaktor verbundene Vorlage portionsweise in die organische Lösung über eine flexible Schlauchverbindung eingetragen, worauf eine exotherme Reaktion in der organischen Lösung erfolgte. Die Zugabe des elementaren Antimons erfolgte derart, daß binnen etwa 2 Stunden etwa 15 Gew.-% der Gesamtmenge des Antimons und nach weiteren 4 Stunden 60 Gew.-% zugegeben waren. Nach einer Gesamtzeit von 6 Stunden war das Antimon vollständig zugegeben. Nach beendeter Antimonzugabe wurde die Lösung noch 5 Stunden bei einer Temperatur von 70 °C gehalten. Vor dem Abkühlen zwecks Kristallisaton wurde der Lösung etwa 100 ml Tetrachlorkohlenstoff zugesetzt. Anschließend ließ man den Reaktor mit Inhalt über Nacht auf Umgebungstemperatur abkühlen. Während des Abkühlvorgangs kristallisierte Antimontrichlorid an der Reaktorwandung aus. In den Reaktor wurde ein Tauchrohr eingeschoben und die flüssige Phase abgesaugt (abgesaugte Menge: 337 g). Der kristalline Rückstand im Reaktor wurde mit 50 ml Tetrachlorkohlenstoff versetzt, aufgeschmolzen und erneut durch Abkühlen auf Umgebungstempertur auskristallisiert. Wiederum wurde die flüssige Phase mittels eines in den Reaktor geschobenen Tauchrohres abgesaugt (abgesaugte Menge: 84 g).

Das im Reaktor verbliebene kristalline Antimontrichlorid besaß einen Reinheitsgrad von 99 %. Die Ausbeute betrug 92 % d.Th.

c) Weiterverarbeitung des aus der organischen Lösung abgetrennten Antimontrichlorids zu Antimonpentachlorid durch Chlorieren:

Das in Stufe (b) isolierte kristalline Antimontrichlorid wurde im Reaktor mit 100 ml Chloroform versetzt. Durch ein Tauchrohr wurde Chlorgas in die flüssige Phase eingeleitet. Die Temperatur der Lösung, die anfangs bei etwa 20°C lag, wurde durch die Reaktionswärme und zusätzliches Heizen allmählich auf etwa 76°C gebracht. Die Chlorierung wurde abgebrochen, als im nachgeschalteten Gaswäscher, der Kaliumiodid enthielt, elementares Chlor nachweisbar wurde. Nach dem Chlorieren wurde der flüssige Reaktorinhalt wiederum mit einem Tauchrohr abgesaugt. Es wurden 1202 g einer Lösung erhalten, welche im wesentlichen aus Antimonpentachlorid (Gehalt: 76,2 Gew.-%) und Chloroform (Gehalt: 23,6 Gew.-%) bestand. Der Gehalt an Verunreinigungen, vorwiegend durch dreiwertiges Antimon gebildet, lag unter 0,2 Gew.-%.

Die hochreine Lösung eignete sich hervorragend als Katalysator für Fluorierungsverfahren mittels Fluorwasserstoff, insbesondere für die Fluorierung von Chloroform mittels wasserfreiem Fluorwasserstoff.

**Patentansprüche**

1. Verfahren zur Aufarbeitung von inaktiven, Antimonhalogenidkatalysatoren und organische Verunreinigungen enthaltenden organischen Lösungen ohne Zugabe von Wasser, dadurch gekennzeichnet, daß man zu dieser Lösung elementares Antimon hinzufügt und die organischen Verunreinigungen vom gebildeten kristallinen Antimontrichlorid abtrennt.

2. Verfahren nach Anspruch 1, dadurch gekennzeichnet, daß man unter Ausschluß von Feuchtigkeit arbeitet.

3. Verfahren nach einem der vorhergehenden Ansprüche, dadurch gekennzeichnet, daß man, während man elementares Antimon zur Lösung hinzufügt, die Lösung bei einer solchen Temperatur hält, daß gebildetes Antimontrichlorid nicht auskristallisiert.

4. Verfahren nach einem der vorhergehenden Ansprüche, dadurch gekennzeichnet, daß man die Lösung während der Umsetzung des elementaren Antimons mit dem fünfwertigen Antimon bei einer Temperatur hält, daß gebildetes Antimontrichlorid nicht auskristallisiert.

5. Verfahren nach Anspruch 3 oder 4, dadurch gekennzeichnet, daß man die Lösung bei Temperaturen zwischen etwa 65 und 100 °C hält.

6. Verfahren nach einem der vorhergehenden Ansprüche, dadurch gekennzeichnet, daß man das Verfahren bei einem Druck von 0,1 bis 6 Bar, vorzugsweise etwa 0,5 bis 3 Bar durchführt.

7. Verfahren nach einem der vorhergehenden Ansprüche, dadurch gekennzeichnet, daß man pro Mol Antimonpentachlorid der verbrauchten Katalysatorlösung etwa 0,5 bis etwa 1 Mol Antimon, vorzugsweise etwa 0,6 bis etwa 0,9 Mol Antimon einsetzt.

8. Verfahren nach einem der vorhergehenden Ansprüche, dadurch gekennzeichnet, daß man das auskristallisierte Antimontrichlorid einer Nachreinigung unterwirft.

9. Verfahren nach einem der vorhergehenden Ansprüche, dadurch gekennzeichnet, daß man das gewünschtenfalls nachgereinigte Antimontrichlorid mit Chlor zu Antimonpentachlorid umsetzt.

10. Verfahren nach Anspruch 9, dadurch gekennzeichnet, daß man die Chlorierung in einem halogenierten organischen Lösungsmittel durchführt.

11. Verfahren nach einem der vorhergehenden Ansprüche, dadurch gekennzeichnet, daß man inaktive, Antimonhalogenidkatalysator enthaltende Lösungen einsetzt, wie sie bei der Fluorierung von halogenierten, insbesondere chlorierten Kohlenwasserstoffen mit einem oder mehreren C-Atomen mit wasserfreiem Fluorwasserstoff anfallen.

## Claims

1. A method for working up inactive organic solutions containing antimony halide catalysts and organic impurities without the addition of water, characterised in that elemental antimony is added to this solution and the organic impurities are separated off from the resulting crystalline antimony trichloride.

2. A method according to Claim 1, characterised in that one works with moisture excluded.

3. A method according to one of the preceding Claims, characterised in that while elemental antimony is being added to the solution the solution is kept at a temperature such that the resulting antimony trichloride does not crystallise out.

4. A method according to one of the preceding Claims, characterised in that during the reaction of the elemental antimony with the pentavalent antimony the solution is kept at a temperature [such] that the resulting antimony trichloride does not crystallise out.

5. A method according to Claim 3 or 4, characterised in that the solution is kept at temperatures of between approximately 65 and 100°C.

6. A method according to one of the preceding Claims, characterised in that the method is carried out at a pressure of 0.1 to 6 bar, preferably about 0.5 to 3 bar.

7. A method according to one of the preceding Claims, characterised in that about 0.5 to about 1 mole antimony, preferably about 0.6 to about 0.9 mole antimony, are used per mole antimony pentachloride of the spent catalyst solution.

8. A method according to one of the preceding Claims, characterised in that the antimony trichloride which has crystallised out is subjected to subsequent purification.

9. A method according to one of the preceding Claims, characterised in that the antimony trichloride, which has been subsequently purified if desired, is reacted with chlorine to form antimony pentachloride.

10. A method according to Claim 9, characterised in that the chlorination is performed in a halogenated organic solvent.

11. A method according to one of the preceding Claims, characterised in that inactive solutions containing antimony halide catalyst are used such as are produced upon the fluorination of halogenated, in particular chlorinated, hydrocarbons having one or more C atoms with anhydrous hydrogen fluoride.

## Revendications

1. Procédé de retraitement de catalyseurs inactifs à base d'halogénure d'antimoine et de solutions organiques contenant des impuretés organiques sans addition d'eau, caractérisé en ce qu'on ajoute à cette solution de l'antimoine élémentaire et sépare les impuretés organiques du trichlorure d'antimoine cristallin formé.

2. Procédé selon la revendication 1, caractérisé en ce qu'on opère à l'abri de l'humidité.

3. Procédé selon l'une des revendications précédentes, caractérisé en ce que, pendant l'addition d'antimoine élémentaire à la solution, on maintient la solution à une température telle que le trichlorure d'antimoine formé ne se sépare pas à l'état cristallin.

4. Procédé selon l'une des revendications précédentes, caractérisé en ce qu'on maintient la solution pendant la réaction de l'antimoine élémentaire avec l'antimoine pentavalent à une température telle que le trichlorure d'antimoine formé ne se sépare pas à l'état cristallin.

5. Procédé selon la revendication 3 ou 4, caractérisé en ce qu'on maintient la solution à des températures entre environ 65 et 100°C.

6. Procédé selon l'une des revendications précédentes, caractérisé en ce qu'on conduit le procédé sous une pression de 0,1 à 6 bars, de préférence d'environ 0,5 à 3 bars.

7. Procédé selon l'une des revendications précédentes, caractérisé en ce qu'on met en oeuvre, par mole de pentachlorure d'antimoine de la solution de catalyseur usée, environ 0,5 à environ 1 mole d'antimoine, de préférence environ 0,6 à environ 0,9 mole d'antimoine.

8. Procédé selon l'une des revendications précédentes, caractérisé en ce qu'on soumet le trichlorure d'antimoine séparé par cristallisation à une purification ultérieure.

9. Procédé selon l'une des revendications précédentes, caractérisé en ce qu'on transforme le trichlorure d'antimoine au besoin postpurifié avec du chlore en pentachlorure d'antimoine.

10. Procédé selon la revendication 9, caractérisé en ce qu'on effectue la chloration dans un solvant organique halogéné.

11. Procédé selon l'une des revendications précédentes, caractérisé en ce qu'on met en oeuvre des solutions inactives contenant du catalyseur à base d'halogénure d'antimoine, telles qu'elles se forment lors de la fluoration d'hydrocarbures à un ou plusieurs atomes de C halogénés, en particulier chlorés, avec de l'acide fluorhydrique anhydre.